# EUROPEAN PATENT SPECIFICATION

(11) **EP 3 222 539 B1**
(45) Date of publication and mention of the grant of the patent: **06.01.2021**
(21) Application number: 15860506.3
(22) Date of filing: 17.11.2015
(51) Int. Cl.: A61L 2/08, B65B 55/08, B65B 55/04, G21K 5/10

(54) **ELECTRON BEAM STERILIZATION DEVICE**
ELEKTRONENSTRAHLSTERILISATIONSVORRICHTUNG
DISPOSITIF DE STÉRILISATION À FAISCEAU D'ÉLECTRONS

(30) Priority: 18.11.2014 JP 2014233189
(43) Date of publication of application: 27.09.2017
(73) Proprietor: Hitachi Zosen Corporation, Osaka-shi, Osaka 559-8559 (JP)
(72) Inventor: SASAKI Kazuaki, Osaka-shi, Osaka 559-8559 (JP); YOKOBAYASHI Takayasu, Osaka-shi, Osaka 559-8559 (JP); TAKEDA Shinichi, Osaka-shi, Osaka 559-8559 (JP)
(74) Representative: Engelhardt, Harald
(86) International application number: PCT/JP2015/082181
(87) International publication number: WO 2016/080369

(56) References cited:
- EP-A1- 2 769 922
- WO-A1-2015/059959
- WO-A1-2015/124357
- JP-A- 2006 314 407
- JP-A- 2014 028 659
- US-A- 5 554 856
- US-A1- 2012 273 694

## Description

The present invention relates to an electron beam sterilization device for applying an electron beam onto an inner surface of containers for foods, liquid drugs or the like being conveyed, thereby to sterilize the containers.

An electron beam sterilization device according to the preamble of claim 1 is known from EP 2 769 922. This electron beam sterilization device includes an outer-surface sterilization zone for sterilizing the outer surface of a container by emitting electron beams when the container is transported along circular paths and an inner-surface sterilization zone for sterilizing the inner surface of the container. An electron beam irradiator is located near the upstream side of a joint of the circular paths disposed in the outer-surface sterilization zone, and another electron beam irradiator is located near the downstream side of the joint. Thus, the electron beam irradiators are brought close to each other so as to sequentially sterilize the one half outer surfaces and the other half outer surface of the container. The sterilization chamber in which the electron beams from the electron beam irradiators are applied to the containers is covered by a blocking member to block radiations.

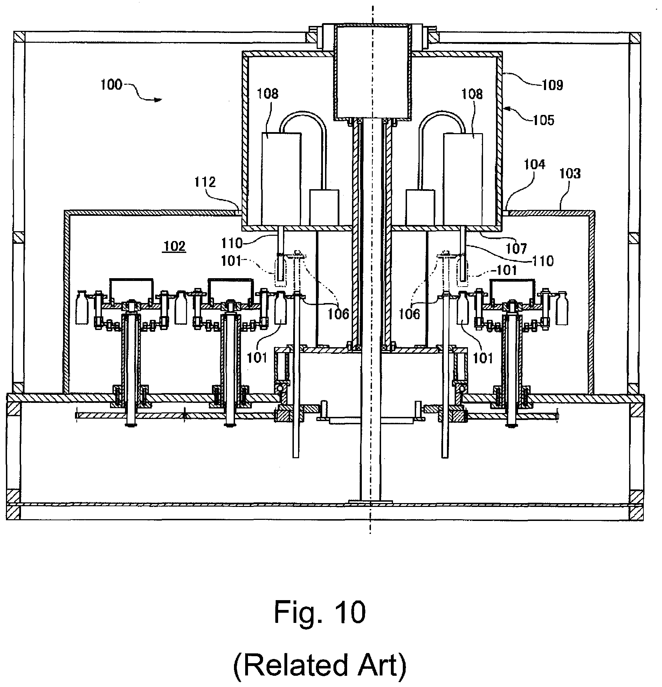

A conventionally configured electron beam sterilization device is shown in Fig. 10. This device includes a sterilization chamber 102 in which electron beams are applied to containers 101 to sterilize the interiors of the containers 101, a blocking cover member 103 that covers the sterilization chamber 102 and blocks radiations, and a rotational member 105 having an upper portion projected through an opening portion 104 formed in the blocking cover member 103.

The rotational member 105 includes a plurality of clamp units 106 configured to retain the containers 101 and capable of elevation, a rotational table 107, a plurality of electron beam emission units 108 provided on the rotational table 107, and a rotational blocking cover 109 that covers the electron beam emission units 108. The electron beam emission units 108 apply electron beams to the containers 101 retained by the clamp units 106 and have nozzles 110. The electron beams are emitted from emission windows at the lower ends of the nozzles 110.

The rotational blocking cover 109 may be provided on the upper surface of the rotational table 107. An upper portion of the blocking cover member 103 has an opening portion 104 formed therein, and an upper portion of the rotational member 105, that is, the rotational blocking cover 109 is exposed upward through the opening portion 104.

In this arrangement, when the rotational member 105 rotates and the clamp units 106 retaining the containers 101 are elevated, the containers 101 are elevated so as to insert the nozzles 110 of the electron beam emission units 108 into the containers 101, and electron beams are emitted from the emission windows of the nozzles 110 to sterilize the interiors of the containers 101.

When the electron beams strike a metal member made of a stainless steel or the like such as the rotational member 105, radiations such as X-rays are generated. These radiations are blocked by the blocking cover member 103.

The electron beam sterilization devices 100 as described above are disclosed in, for example, JP 2013 86822 A.

However, in the above conventional arrangement, the radiations generated in the sterilization chamber 102 may leak outside through the gap 112 between the opening portion 104 and the rotational blocking cover 109. One object of the present invention is to provide an electron beam sterilization device in which the radiations generated in the sterilization chamber can be blocked reliably.

To achieve the above object, an electron beam sterilization device according to the first aspect of the present invention comprises a Plurality of electron beam emission units configured to emit electron beam, a sterilization chamber in which electron beams from the electron beam emission units are applied to containers to sterilize at least part of the containers; a blocking cover member covering the sterilization chamber so as to block radiations; and a rotational member having an upper portion thereof exposed through an opening portion formed in the blocking cover member, wherein the blocking cover member includes a fixed blocking cover covering the upper portion of the rotational member, an air passing space is formed between the fixed blocking cover and the upper portion of the rotational member, between the rotational member and the fixed blocking cover, there are provided a rotational annular blocking plate and a fixed annular blocking plate, the rotational annular blocking plate projecting from the rotational member toward the fixed blocking cover, the fixed annular blocking plate projecting from the blocking cover member toward the rotational member, the rotational annular blocking plate and the fixed annular blocking plate are opposed to each other with a blocking gap maintained therebetween, and the rotational annular blocking plate and the fixed annular blocking plate constitute a blocking lap portion for blocking radiations.

In this arrangement, in the sterilization chamber, the rotational member rotates and electron beams are applied to the containers to sterilize the containers. The radiations generated in the sterilization chamber are blocked by the blocking cover member. In addition, the radiations in the sterilization chamber are blocked by the blocking lap portion, and therefore, only an extremely small amount of radiations travels out of the sterilization chamber through the blocking lap portion and enters the air passing space. Further, the radiations entering the air passing space are blocked by the fixed blocking cover of the blocking cover member, and therefore, the radiations can be blocked reliably.

In the electron beam sterilization device according to a second aspect of the present invention, the rotational member includes a rotational table and a rotational blocking cover, the rotational table having the plurality of electron beam emission units arranged thereon in a circumferential direction, the rotational blocking cover being provided on an upper surface of the rotational table so as to cover the electron beam emission units, the rotational blocking cover is exposed through an opening portion of the blocking cover member and covered by the fixed blocking cover, and in the sterilization chamber, nozzles project from the electron beam emission units through the rotational table, and the electron beam sterilization device is configured to insert the nozzles into the containers and emit electron beams from the nozzles to sterilize at least inner surfaces of the containers.

In the electron beam sterilization device according to a third aspect of the present invention, blocking members incorporated into the rotational annular blocking plate and the fixed annular blocking plate have a thickness larger than that of a blocking member incorporated into the fixed blocking cover.

In this arrangement, only an extremely small amount of radiations travels out of the sterilization chamber through the blocking lap portion and enter the air passing space. In the electron beam sterilization device according to a fourth aspect of the present invention, the fixed blocking cover has an air inlet for delivering a scavenging air through the air passing space and the blocking gap to the sterilization chamber.

In this arrangement, when the scavenging air is fed through the air inlet into the fixed blocking cover, the scavenging air flows through the air passing space and the blocking gap and enters the sterilization chamber. During the sterilization process, application of electron beams to the containers produces ozone gas in the sterilization chamber. Since the scavenging air flows from the air inlet through the air passing space end enters the sterilization chamber, the ozone gas in the sterilization chamber can be prevented from flowing through the blocking gap and entering the space between the fixed blocking cover and the upper portion of the rotational member.

### ADVANTAGES

According to the present invention as described above, the radiations generated in the sterilization chamber are blocked by the blocking lap portion, and therefore, only an extremely small amount of radiations travels out of the sterilization chamber through the blocking lap portion and enters the air passing space. Further, the radiations entering the air passing space are blocked by the fixed blocking cover of the blocking cover member, and therefore, the radiations can be blocked reliably.

### BRIEF DESCRIPTION OF THE DRAWINGS

Fig. 1 is a longitudinal sectional view of an electron beam sterilization device according to an embodiment of the present invention.
Fig. 2 shows a plastic bottle to be sterilized by the electron beam sterilization device of the embodiment.
Fig. 3 is a schematic plan view of a plurality of chambers arranged in the electron beam sterilization device of the embodiment.
Fig. 4 is a sectional view of a clamp unit, an elevation unit, and an opening/closing mechanism of the electron beam sterilization device of the embodiment.
Fig. 5 is a view along the line X-X in Fig. 1.
Fig. 6 is a plan view of the clamp unit of the electron beam sterilization device of the embodiment.
Fig. 7 is an enlarged sectional view of a blocking lap portion of the electron beam sterilization device of the embodiment.
Fig. 8 is a view along the line X-X in Fig. 7.
Fig. 9 shows a preform product to be sterilized by the electron beam sterilization device of the embodiment.
Fig. 10 is a longitudinal sectional view of a conventional electron beam sterilization device.

### DESCRIPTION OF EXAMPLE EMBODIMENTS

Embodiments of the present invention will be hereunder described with reference to the accompanying drawings.

### First Embodiment

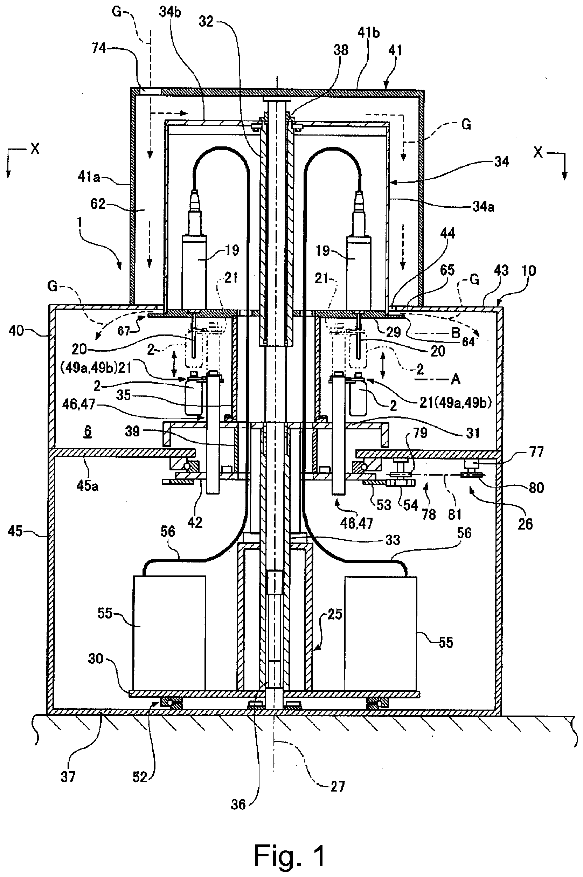

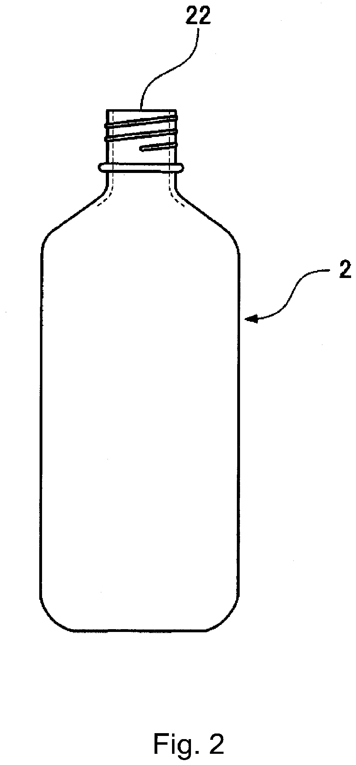

In the first embodiment, an electron beam sterilization device 1 may apply electron beams to plastic bottles 2 (an example of bottles) for sterilization, as shown in Figs. 1 and 2. The plastic bottles 2 may be formed from a preform product having a test tube shape (with a U-shaped longitudinal section opened at the upper end thereof) subjected to blow molding.

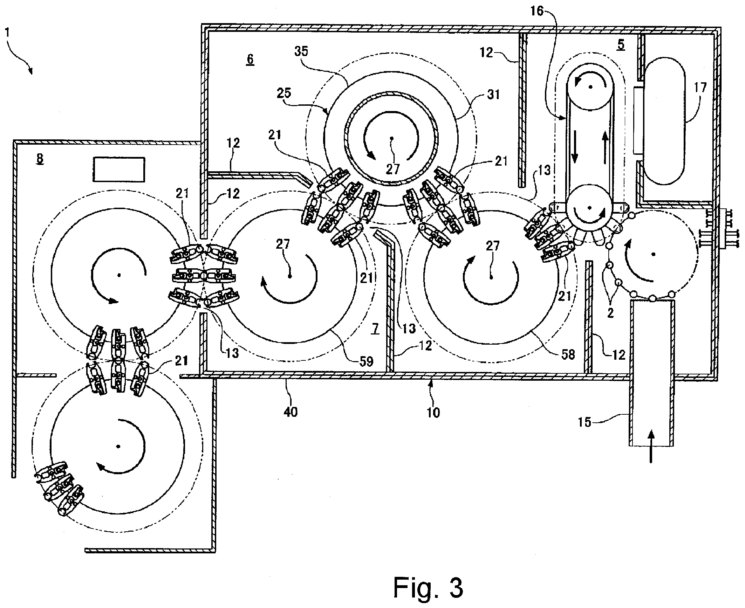

As shown in Fig. 3, the electron beam sterilization device 1 may include four chambers 5 to 8 and a blocking cover member 10 that covers the chambers 5 to 8 and blocks radiations. The chambers 5 to 8, which may be arranged from the upstream side of the conveyance path of the plastic bottles 2, may include an outer surface sterilization chamber 5 in which electron beams are applied to outer surfaces of the plastic bottles 2 fed from outside so as to perform sterilization, an inner surface sterilization chamber 6 in which electron beams are applied to inner surfaces of the plastic bottles 2 so as to perform sterilization, a blocking chamber 7 for blocking radiations generated by application of the electron beams to the plastic bottles 2, and a sorting chamber 8 in which the plastic bottles 2 that are insufficiently sterilized (hereinafter referred to simply as "defective products") are ejected. The chambers 5 to 8 may be separated by partition walls 12. Each of the partition walls 12 may have a communication hole 13 that accomplish communication between adjacent ones of the chambers 5 to 8.

The outer surface sterilization chamber 5 may include an input portion 15 through which plastic bottles 2 are put in from outside, a conveyor portion 16 for conveying the plastic bottles 2 having been put in, and an outer-surface electron beam emission unit 17 for applying electron beams to outer surfaces of the plastic bottles 2 being conveyed by the conveyor portion 16.

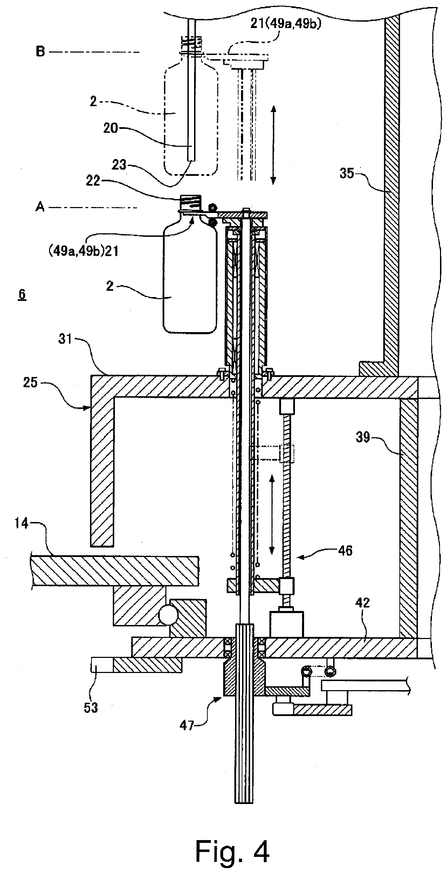

As shown in Figs. 1 and 4, in the inner surface sterilization chamber 6, the plastic bottles 2 retained by the clamp units 21 (an example of the retaining devices) may be moved vertically with respect to the nozzles 20 of a plurality of electron beam emission units 19, so as to insert the nozzles 20 into the plastic bottles 2 through the opening portions 22, and electron beams may be emitted from emission windows 23 of the nozzles 20 to sterilize the interiors of the plastic bottles 2.

The inner surface sterilization chamber 6 may include the rotational member 25 that may be rotationally driven around a vertical rotational axis 27 by the rotational drive unit 26. The rotational member 25 may include an upper rotational table 29 (an example of rotational table), a lower rotational table 30, an intermediate rotational table 31 provided between the upper rotational table 29 and the lower rotational table 30, first and second rotational shafts 32, 33 in a vertical arrangement along the rotational axis 27, a rotational blocking cover 34 (an example of the upper portion of the rotational member), a blocking cylinder 35 having a circular shape and provided between the upper rotational table 29 and the intermediate rotational table 31, a mounting cylinder 39 having a circular shape and provided on the lower surface of the intermediate rotational table 31, and a mounting table 42 having a ring shape and provided on the lower end of the mounting cylinder 39.

A fixed shaft 36 may extend through the first and second rotational shafts 32, 33 and may be mounted to a bottom frame plate 37. Both the first and second rotational shafts 32, 33 may be rotatably supported by the fixed shaft 36 via a bearing 38.

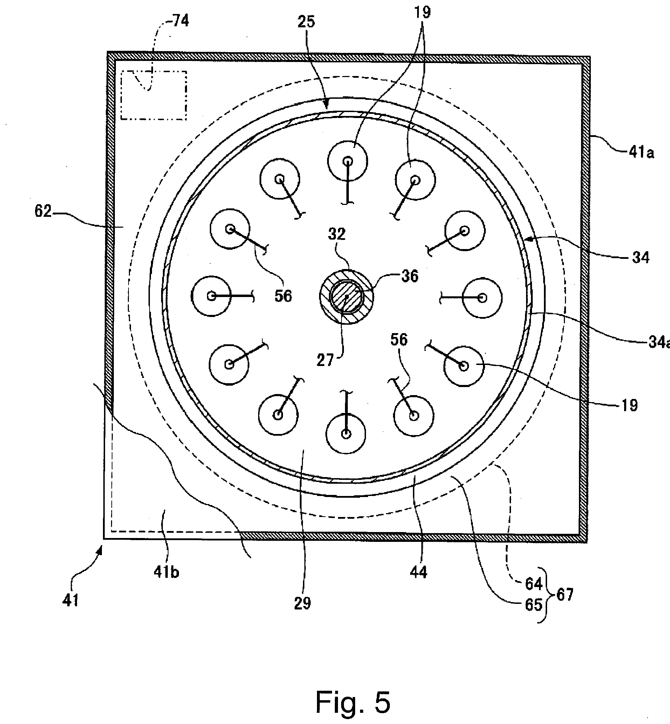

As shown in Figs. 1 and 5, the upper rotational table 29 may have a disk shape. Electron beam emission units 19, each projecting the nozzle 20 downward, may be arranged on the outer peripheral portion of the upper rotational table 29 at regular angular intervals.

The rotational blocking cover 34 may be provided on the upper surface of the upper rotational table 29 and may cover all the electron beam emission units 19. The blocking cover member 10 may include a cover body 40, a fixed blocking cover 41, and a support frame 45 that supports the cover body 40. The cover body 40 may include an upper cover plate 43 that extends over the chambers 5 to 8. The cover body 40 may be removably mounted on the support frame 45. The support frame 45 may include a ceiling frame 45a at the upper end thereof. The ceiling frame 45a may have a horizontal plate shape and may be projected inside the blocking cover member 10. In the inner surface sterilization chamber 6, the upper cover plate 43 may have a circular opening portion 44 formed therein.

The rotational blocking cover 34 may be inserted into the opening portion 44 from below so as to be exposed above the upper cover plate 43 and covered by the fixed blocking cover 41. The rotational blocking cover 34 may include a circular barrel cover 34a and a circular ceiling cover 34b. The circular barrel cover 34a may have a cylindrical shape and may be provided on the upper rotational table 29, and the circular ceiling cover 34b may have a disk shape and may close the upper end of the circular barrel cover 34a.

The inner surface sterilization chamber 6 may be formed below the upper rotational table 29. In the inner sterilization chamber 6, the clamp units 21 may be provided in the outer peripheral portion of the intermediate rotational table 31 and arranged below the nozzles 20 of the electron beam emission units 19 so as to be opposed thereto. As shown in Figs. 1 and 4, on the outer peripheral portion of the intermediate rotational table 31, there may be provided a plurality of elevation units 46 for elevating and lowering the clamp units 21, and opening/closing mechanisms 47.

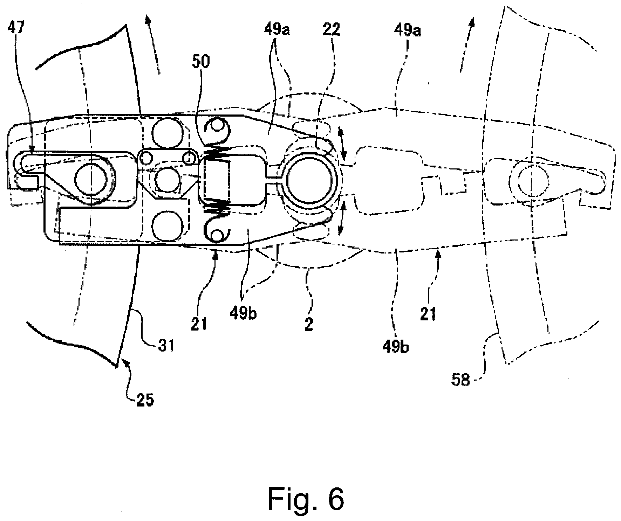

As shown in Figs. 6, each of the clamp units 21 may include a pair of clamp arms 49a, 49b capable of opening and closing and a spring 50 (an example of biasing member) that biases the pair of clamp arms 49a, 49b toward the respective closing directions. The opening/closing mechanism 47 may operate to open and close the clamp arms 49a, 49b.

As shown in Fig. 1, the lower rotational table 30 may have a disk shape and may be rotatably supported on the bottom frame plate 37 via a support member 52. On the lower rotational table 30, there may be provided a plurality of power supply units 55 for supplying power to the electron beam emission units 19. The electron beam emission units 19 and the power supply units 55 may be connected via the power supply cables 56.

A ring gear 53 may be provided in the outer periphery of the mounting table 42 over the whole circumference thereof. The rotational drive unit 26 may include a drive gear 54 meshing with the ring gear 53, a motor 77, a transmission mechanism 78 that transmits the rotation of the motor 77 to the drive gear 54. The transmission mechanism 78 may include a pair of sprockets 79, 80 and a chain 81 wound around the sprockets 79, 80. The drive gear 54 and the motor 77 may be provided on the ceiling frame 45a of the support frame 45.

As shown in Fig. 3, on one side of the rotational member 25, there may be provided a turn rotation table 58 for receiving plastic bottles 2 conveyed from the outer surface sterilization chamber 5 and conveying them to the clamp units 21 of the rotational member 25. Further, on the other side of the rotational member 25, there may be provided a sterilized rotation table 59 for receiving the plastic bottles 2 from the rotational member 25.

Both the rotation tables 58, 59 may be rotationally driven around the respective vertical rotational axes 27, as with the rotational member 25. As with the rotational member 25, each of the rotation tables 58, 59 may be provided with a plurality of clamp units 17. While the rotational member 25 and the rotation tables 58, 59 rotate, the plastic bottles 2 can be passed from the clamp units 21 of the turn rotation table 58 to the clamp units 21 of the rotational member 25, and the plastic bottles 2 can also be passed from the clamp units 21 of the rotational member 25 to the clamp units 21 of the sterilized rotation table 59.

As shown in Figs. 1 and 5, the fixed blocking cover 41 may include a rectangular barrel cover 41a and a rectangular ceiling cover 41b. The rectangular barrel cover 41a may have a rectangular tube shape and may be provided on the upper cover plate 43 of the cover body 40, and the rectangular ceiling cover 41b may have a rectangular plate shape and may close the upper end of the rectangular barrel cover 41a. Further, an air passing space 62 may be formed between the fixed blocking cover 41 and the rotational blocking cover 34.

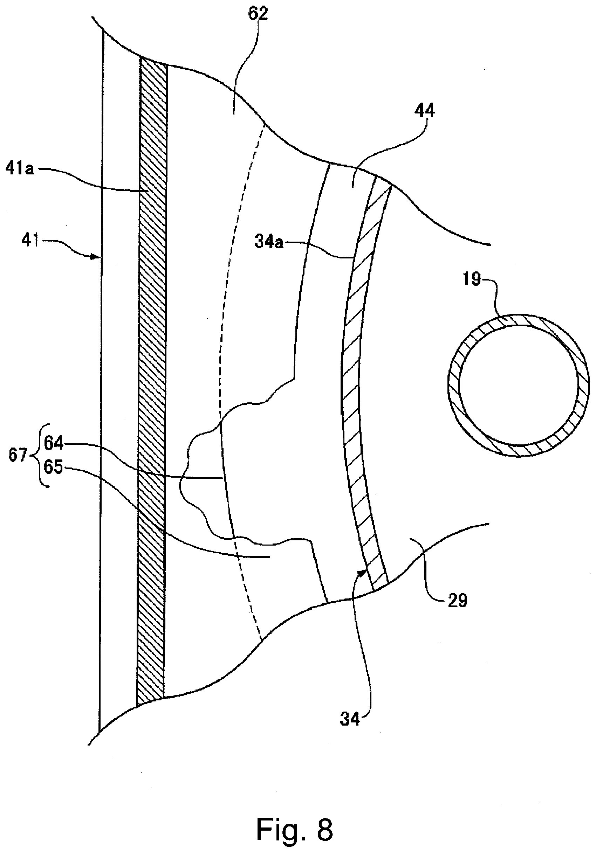

As shown in Figs. 1, 5, 7 and 8, on the outer peripheral portion of the upper rotational table 29, a rotational annular blocking plate 64 may be provided over the whole circumference so as to project toward the fixed blocking cover 41 (i.e., toward the outer periphery) by a constant width. Also, on the upper cover plate 43 of the cover body 40 of the blocking cover member 10, a fixed annular blocking plate 65 may be provided over the whole circumference so as to project toward the rotational blocking cover 34 of the rotational member 25 (i.e., toward the inner periphery). In addition, the fixed annular blocking plate 65 may encircle the lower end portion of the rotational blocking cover 34, and the inner periphery of the fixed annular blocking plate 65 may face the opening portion 44.

The fixed annular blocking plate 65 may be positioned above the rotational annular blocking plate 64, and the rotational annular blocking plate 64 and the fixed annular blocking plate 65 may be vertically opposed to each other with a blocking gap 66 maintained therebetween. The blocking gap 66 may be formed between the rotational annular blocking plate 64 and the fixed annular blocking plate 65 over the whole circumference and may communicate with the inner surface sterilization chamber 6 and the air passing space 62. The rotational annular blocking plate 64 and the fixed annular blocking plate 65 may constitute a blocking lap portion 67 that blocks radiations.

The cover body 40, the fixed blocking cover 41, and the fixed annular blocking plate 65 of the blocking cover member 10, and the upper rotational table 29 and the rotational annular blocking plate 64 of the rotational member 25 may be made of a corrosion-resistant metal such as a stainless steel. Further, the cover body 40, the fixed blocking cover 41, the rotational annular blocking plate 64, and the fixed annular blocking plate 65 may internally have blocking members 69, 70, 71, and 72, respectively. These blocking members may be made of lead or the like and may block the radiations. Likewise, the ceiling frame 45a of the support frame 45 may also have a blocking member provided therein.

Among these blocking members, the blocking members 71, 72 incorporated into the rotational annular blocking plate 64 and the fixed annular blocking plate 65 may have a thickness T larger than that (a thickness t) of the blocking member 70 incorporated into the fixed blocking cover 41. As shown in Figs. 1 and 3, the rectangular ceiling cover 41b of the fixed blocking cover 41 may have an air inlet 74 provided therein. The air inlet 74 may serve to deliver a clean air G (an example of scavenging air) having a positive pressure (i.e., a pressure equal to or greater than 1 atm) from the air passing space 62 through the blocking gap 66 to the inner surface sterilization chamber 6.

The operations in the above arrangement will now be described. When the motor 77 drives, the rotation of the motor 77 may be transmitted to the ring gear 53 on the mounting table 42 via the transmission mechanism 78, and the rotational member 25 may be rotated. As shown in Fig. 3, while the turn rotation table 58, the rotational member 25, and the sterilized rotation table 59 rotate, and the plastic bottles 2 are put into the outer surface sterilization chamber 5 and conveyed, electron beams may be applied from the outer-surface electron beam emission unit 17 to the outer surfaces of the plastic bottles 2, thereby to sterilize the outer surfaces of the plastic bottles 2. The rotation of the rotational member 25 may be integrated with the rotation of the rotational blocking cover 34, the clamp units 21, the elevation units 46, the opening/closing mechanisms 47, the electron beam emission units 19, the power supply units 55, and the power supply cables 56.

Then, the plastic bottles 2 may be conveyed from the outer surface sterilization chamber 5, retained by the clamp units 21 of the turn rotation table 58 of the inner surface sterilization chamber 6, and passed from the clamp units 21 of the turn rotation table 58 to the clamp units 21 of the rotational member 25. At this time, as shown in Fig. 6, the opening/closing mechanisms 47 may cause the clamp arms 49a, 49b of the turn rotation table 58 to switch from the closed state and open, while the clamp arms 49a, 49b of the rotational member 25 may switch from the opened state and close, whereby the plastic bottles 2 may be retained by the clamp units 21 of the rotational member 25.

As represented by the virtual lines in Figs. 1 and 4, the elevation unit may elevate the clamp arms 49a, 49b from the lowest position A to the highest position B, the plastic bottle 2 retained by the clamp arms 49a, 49b may be elevated to the highest position B, such that the nozzle 20 of the electron beam emission unit 19 is inserted into the plastic bottle 2 through the opening portion 22 thereof. The interior of the plastic bottle 2 may be sterilized by the electron beam emitted from the emission window 23 of the nozzle 20.

Then, as represented by the solid lines in Figs. 1 and 4, the elevation unit 46 may lower the clamp arms 49a, 49b from the highest position B to the lowest position A. Thus, the plastic bottle 2 retained by the clamp arms 49a, 49b may be lowered to the lowest position A, such that the nozzle 20 of the electron beam emission unit 19 is separated upward from the plastic bottle 2.

Then, as shown in Fig. 3, the plastic bottles 2 may be passed from the clamp units 21 of the rotational member 25 to the clamp units 21 of the sterilized rotation table 59. At this time, the opening/closing mechanism 47 may cause the clamp arms 49a, 49b of the rotational member 25 to switch from the closed state and open, while the clamp arms 49a, 49b of the sterilized rotation table 59 may switch from the opened state and close, whereby the plastic bottles 2 may be retained by the clamp units 21 of the sterilized rotation table 59.

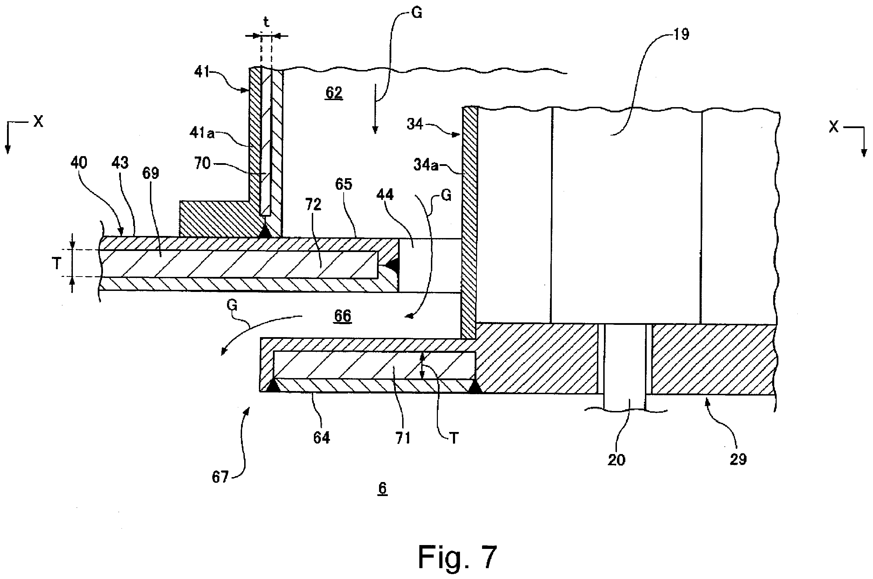

During this sterilization process, the radiations generated in the outer surface sterilization chamber 5 and the inner surface sterilization chamber 6 may be blocked by the blocking cover member 10. As shown in Figs. 1 and 7, the radiations in the inner surface sterilization chamber 6 may be blocked by the blocking lap portion 67, and therefore, only an extremely small amount of radiations may travel out of the inner surface sterilization chamber 6 through the blocking lap portion 67 and enter the air passing space 62. Further, the radiations entering the air passing space 62 may be blocked by the fixed blocking cover 41, and therefore, the radiations can be blocked reliably.

In addition, the electron beams emitted from the nozzles 20 of the electron beam emission units 19 may react with oxygen in the air to produce ozone in the inner surface sterilization chamber 6. To address the production of ozone, a clean air G (hereinafter referred to as the scavenging air) having a positive pressure may be supplied to the air inlet 74, as shown in Figs. 1 and 7. The scavenging air G supplied into the fixed blocking cover 41 through the air inlet 74 may flow through the air passing space 62, enter the inner surface sterilization chamber 6 through the blocking gap 66, flow in the inner surface sterilization chamber 6, and then flow into the outer surface sterilization chamber 5 through the communication hole 13.

Thus, the ozone gas in the inner surface sterilization chamber 6 may flow out to the outer surface sterilization chamber 5 along with the scavenging air G, and therefore, the ozone gas produced in the inner surface sterilization chamber 6 can be prevented from entering (flowing into) the air passing space 62 through the blocking gap 66 and accumulating there.

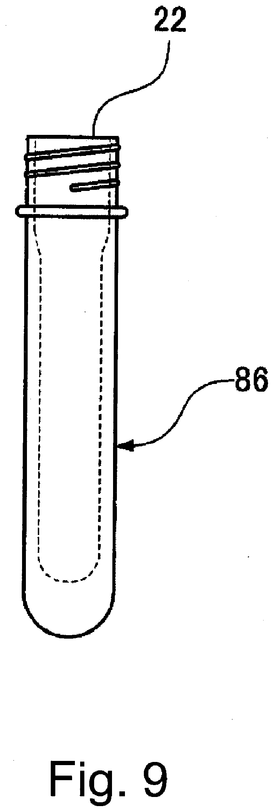

In the above embodiment, a clean air G may be used as an example of the scavenging air. It may also be possible to use an inert gas such as nitrogen gas. As shown in Fig. 2, the above embodiment is used for plastic bottles 2 as an example of containers. However, the containers are not limited to the plastic bottles 2. For example, it may also be possible that, as shown in Fig. 9, the containers are preform products 86 prior to blow molding, bags, or the like.

In the above embodiment, as shown in Figs. 1 and 4, the electron beam emission units 19 may be fixed in the vertical direction, and the plastic bottles are elevated and lowered by the elevation units 46, so as to insert the nozzles 20 of the electron beam emission units 19 into the plastic bottles 2 and pull it out. It may also be possible that the plastic bottles 2 are fixed in the vertical direction, and the electron beam emission units 19 are elevated and lowered by the elevation units 46, so as to insert the nozzles 20 of the electron beam emission units 19 into the plastic bottles 2 and pull it out. Further, it may also be possible that both the plastic bottles 2 and the electron beam emission units 19 are elevated and lowered by the elevation units 46, so as to insert the nozzles 20 of the electron beam emission units 19 into the plastic bottles 2 and pull it out.

In the electron beam sterilization device 1 of the above embodiment, the outer surfaces of the plastic bottles 2 are sterilized in the outer surface sterilization chamber 5, and the inner surfaces of the plastic bottles 2 are sterilized in the inner surface sterilization chamber 6. It may also be possible that only one of the outer surfaces and the inner surfaces of the plastic bottles 2 are sterilized.

In the above embodiment, the scavenging air G supplied into the fixed blocking cover 41 through the air inlet 74 may flow through the air passing space 62, enter the inner surface sterilization chamber 6 through the blocking gap 66, flow in the inner surface sterilization chamber 6, and then flow into the outer surface sterilization chamber 5 through the communication hole 13. It may also be possible that the cover body 40 has an air outlet formed therein and the scavenging air G flowing in the inner surface sterilization chamber 6 is discharged through the air outlet.

## Claims

1. An electron beam sterilization device (1) comprising:
a plurality of electron beam emission units (19) configured to emit electron beam,
a sterilization chamber (6) in which electron beams from the electron beam emission units (19) are applied to containers (2) to sterilize at least part of the containers (2) and
a blocking cover member (10) covering the sterilization chamber (6) so as to block radiations, **characterized in that**
a rotational member (25) having an upper portion thereof exposed through an opening portion (44) formed in the blocking cover member (10),
wherein the blocking cover member (10) includes a fixed blocking cover (41) covering the upper portion of the rotational member (25),
an air passing space (62) is formed between the fixed blocking cover (41) and the upper portion of the rotational member (25),
between the rotational member (25) and the fixed blocking cover (41), there are provided a rotational annular blocking plate (64) and a fixed annular blocking plate (65), the rotational annular blocking plate (64) projecting from the rotational member (25) toward the fixed blocking cover (41), the fixed annular blocking plate (65) projecting from the blocking cover member (10) toward the rotational member (25),
the rotational annular blocking plate (64) and the fixed annular blocking plate (65) are opposed to each other with a blocking gap (66) maintained therebetween, and
the rotational annular blocking plate (64) and the fixed annular blocking plate (65) constitute a blocking lap portion (67) for blocking radiations.

2. The electron beam sterilization device (1) of claim 1, wherein
the rotational member (25) includes a rotational table (29) and a rotational blocking cover (34), the rotational table (29) having the plurality of electron beam emission units (19) arranged thereon in a circumferential direction, the rotational blocking cover (34) being provided on an upper surface of the rotational table (29) so as to cover the electron beam emission units (19),
the rotational blocking cover (34) is exposed through an opening portion (44) of the blocking cover member (10) and covered by the fixed blocking cover (41), and
in the sterilization chamber (6), nozzles (20) project from the electron beam emission units (19) through the rotational table (29), and the electron beam sterilization device (1) is configured to insert the nozzles (20) into the containers (2) and emit electron beams from the nozzles (20) to sterilize at least inner surfaces of the containers (2).

3. The electron beam sterilization device (1) of claim 1 or 2, wherein blocking members (71, 72) incorporated into the rotational annular blocking plate (64) and the fixed annular blocking plate (65) have a thickness larger than that of a blocking member (70) incorporated into the fixed blocking cover (41).

4. The electron beam sterilization device (1) of claim 1 or 2, wherein the fixed blocking cover (41) has an air inlet (74) for delivering a scavenging air through the air passing space (62) and the blocking gap (66) to the sterilization chamber (6).

## Patentansprüche

1. Elektronenstrahlsterilisationsvorrichtung (1), umfassend:
eine Vielzahl von Elektronenstrahlemissionseinheiten (19), die zum Emittieren eines Elektronenstrahls ausgelegt sind,
eine Sterilisationskammer (6), in der Elektronenstrahle aus den Elektronenstrahlemissionseinheiten (19) auf Behälter (2) angewendet werden, um mindestens einen Teil der Behälter (2) zu sterilisieren, und
ein blockierendes Abdeckelement (10), welches die Sterilisationskammer (6) abdeckt, um so Strahlungen zu blockieren, **dadurch gekennzeichnet, dass** ein rotierendes Element (25) mit einem oberen Anteil davon, der durch einen Öffnungsanteil (44) exponiert wird, der in dem blockierenden Abdeckelement (10) gebildet ist,
wobei das blockierende Abdeckelement (10) eine fixierte blockierende Abdeckung (41) einschließt, die den oberen Anteil des rotierenden Elements (25) bedeckt,
ein Luftpassageraum (62) zwischen der fixierten blockierenden Abdeckung (41) und dem oberen Anteil des rotierenden Elements (25) gebildet ist,
zwischen dem rotierenden Element (25) und der fixierten blockierenden Abdeckung (41) eine rotierende ringförmige blockierende Platte (64) und eine fixierte ringförmige blockierende Platte (65) bereitgestellt werden, wobei die rotierende ringförmige blockierende Platte (64) aus dem rotierenden Element (25) in Richtung der fixierten blockierenden Abdeckung (41) vorspringt, wobei die fixierte ringförmige blockierende Platte (65) aus dem blockierenden Abdeckelement (10) in Richtung des rotierenden Elements (25) vorspringt,
die rotierende ringförmige blockierende Platte (64) und die fixierte ringförmige blockierende Platte (65) einander gegenüber liegen, wobei ein blockierender Spalt (66) zwischen ihnen aufrechtgehalten wird, und
die rotierende ringförmige blockierende Platte (64) und die fixierte ringförmige blockierende Platte (65) einen blockierenden Überlappungsanteil (67) zum Blockieren von Strahlungen bilden.

2. Elektronenstrahlsterilisationsvorrichtung (1) nach Anspruch 1, wobei
das rotierende Element (25) einen rotierenden Tisch (29) und eine rotierende blockierende Abdeckung (34) einschließt, wobei der rotierende Tisch (29) die Vielzahl von Elektronenstrahlemissionseinheiten (19) in Umfangrichtung darauf angeordnet aufweist, wobei die rotierende blockierende Abdeckung (34) auf einer oberen Oberfläche des rotierenden Tisches (29) bereitgestellt wird, um so die Elektronenstrahlemissionseinheiten (19) abzudecken, die rotierende blockierende Abdeckung (34) durch einen Öffnungsanteil (44) des blockierenden Abdeckelements (10) exponiert wird und durch die fixierte blockierende Abdeckung (41) abgedeckt wird, und
in der Sterilisationskammer (6) Düsen (20) aus den Elektronenstrahlemissionseinheiten (19) durch den rotierenden Tisch (29) hindurch vorspringen, und wobei die Elektronenstrahlsterilisationsvorrichtung (1) ausgelegt ist, um die Düsen (20) in die Behälter (2) einzuführen und Elektronenstrahle aus den Düsen (20) zu emittieren, um mindestens die Innenoberflächen der Behälter (2) zu sterilisieren.

3. Elektronenstrahlsterilisationsvorrichtung (1) nach Anspruch 1 oder 2, wobei blockierende Elemente (71, 72), die in die rotierende ringförmige blockierende Platte (64) und die fixierte ringförmige blockierende Platte (65) eingebaut sind, eine Dicke aufweisen, die größer als diejenige eines blockierenden Elements (70) ist, das in die fixierte blockierende Abdeckung (41) eingebaut ist.

4. Elektronenstrahlsterilisationsvorrichtung (1) nach Anspruch 1 oder 2, wobei die fixierte blockierende Abdeckung (41) einen Lufteinlass (74) aufweist, um Spülluft durch den Luftpassageraum (62) und den blockierenden Spalt (66) an die Sterilisationskammer (6) abzugeben.

## Revendications

1. Dispositif de stérilisation à faisceau d'électrons (1) comprenant :
une pluralité d'unités d'émission de faisceau d'électrons (19) configurées pour émettre un faisceau d'électrons,
une chambre de stérilisation (6) dans laquelle des faisceaux d'électrons provenant des unités d'émission de faisceau d'électrons (19) sont appliqués sur des conteneurs (2) pour stériliser au moins une partie des conteneurs (2) et
un élément de couverture de blocage (10) recouvrant la chambre de stérilisation (6) de manière à bloquer les radiations, **caractérisé en ce que**
un élément rotatif (25) dont la partie supérieure est exposée à travers une partie d'ouverture (44) formée dans l'élément de couverture de blocage (10),
l'élément de couverture de blocage (10) comprenant un couvercle de blocage fixe (41) couvrant la partie supérieure de l'élément rotatif (25),
un espace de passage de l'air (62) étant formé entre le couvercle de blocage fixe (41) et la partie supérieure de l'élément rotatif (25),
entre l'élément rotatif (25) et le couvercle de blocage fixe (41), une plaque de blocage annulaire rotative (64) et une plaque de blocage annulaire fixe (65) étant prévues, la plaque de blocage annulaire rotative (64) faisant saillie à partir de l'élément rotatif (25) vers le couvercle de blocage fixe (41), la plaque de blocage annulaire fixe (65) faisant saillie à partir de l'élément de couvercle de blocage (10) vers l'élément rotatif (25),
la plaque de blocage annulaire rotative (64) et la plaque de blocage annulaire fixe (65) étant opposées l'une à l'autre avec un espace de blocage (66) maintenu entre elles, et
la plaque de blocage annulaire rotative (64) et la plaque de blocage annulaire fixe (65) constituant une partie de recouvrement de blocage (67) pour bloquer les radiations.

2. Dispositif de stérilisation à faisceau d'électrons (1) selon la revendication 1, l'élément rotatif (25) comprenant une table rotative (29) et un couvercle de blocage rotatif (34), la table rotative (29) ayant la pluralité d'unités d'émission de faisceau d'électrons (19) agencées sur celle-ci dans une direction circonférentielle, le couvercle de blocage rotatif (34) étant prévu sur une surface supérieure de la table rotative (29) de manière à couvrir les unités d'émission de faisceau d'électrons (19),
le couvercle de blocage rotatif (34) étant exposé à travers une partie d'ouverture (44) de l'élément de couvercle de blocage (10) et couvert par le couvercle de blocage fixe (41), et
dans la chambre de stérilisation (6), des buses (20) faisant saillie à partir des unités d'émission de faisceau d'électrons (19) à travers la table rotative (29), et le dispositif de stérilisation à faisceau d'électrons (1) étant configuré pour insérer les buses (20) dans les récipients (2) et émettre des faisceaux d'électrons à partir des buses (20) pour stériliser au moins des surfaces intérieures des récipients (2).

3. Dispositif de stérilisation à faisceau d'électrons (1) selon la revendication 1 ou 2, des éléments de blocage (71, 72) incorporés dans la plaque de blocage annulaire rotative (64) et la plaque de blocage annulaire fixe (65) ayant une épaisseur supérieure à celle d'un élément de blocage (70) incorporé dans le couvercle de blocage fixe (41).

4. Dispositif de stérilisation à faisceau d'électrons (1) selon la revendication 1 ou 2, le couvercle de blocage fixe (41) ayant une entrée d'air (74) pour délivrer un air de balayage à travers l'espace de passage d'air (62) et l'espace de blocage (66) vers la chambre de stérilisation (6).
